# EUROPÄISCHE PATENTSCHRIFT

(11) **EP 3 936 177 B1**
(45) Veröffentlichungstag und Bekanntmachung des Hinweises auf die Patenterteilung: **30.08.2023**
(21) Anmeldenummer: 20184927.0
(22) Anmeldetag: 09.07.2020
(51) Int. Cl.: A61M 16/00

(54) **BEATMUNGSMASCHINE**
BREATHING APPARATUS
RESPIRATEUR

(43) Veröffentlichungstag der Anmeldung: 12.01.2022
(73) Patentinhaber: Albert, Alfred, 97688 Bad Kissingen (DE)
(72) Erfinder: Laier-Groeneveld, Gerhard, 44789 Bochum (DE)
(74) Vertreter: Götz, Georg Alois

(56) Entgegenhaltungen:
- EP-A1- 2 869 427
- WO-A1-00/21596
- WO-A1-99/08738
- US-A- 2 918 917
- US-A- 5 485 850
- US-A- 5 850 835
- US-A1- 2005 098 179
- US-A1- 2006 005 834

## Beschreibung

Die Erfindung betrifft eine Beatmungsmaschine zur Beatmung eines Patienten, wobei die Beatmungsmaschine eine Überdruckeinheit zum Bereitstellen eines Atemgas-Druckstroms in einem oder für einen Inspirationsschenkel aufweist.

Eine erwachsene Person atmet reflexartig etwa zwölf Mal pro Minute ein und aus. In den Lungenbläschen kommt der Sauerstoff der Atem luft mit Blut in Kontakt, wobei das Blut den Sauerstoff den Organen zuführt.

Der Mensch verbraucht pro Minute etwa 250 Milliliter Sauerstoff und produziert ca 230 Milliliter Kohlenstoffdioxid. Aufgrund der Sauerstoffkonzentration von 21% in der Luft, die durch Zugabe von Sauerstoff noch erhöht werden kann, können mit einem Liter Luft 210ml Sauerstoff aufgenommen werden. Es können jedoch nur 4%, d.h. 40 Milliliter Kohlenstoffdioxid pro Liter Luft abgegeben werden. Daher wird für die Kohlenstoffdioxid-Abgabe geatmet.

Sind Patienten physisch nicht mehr in der Lage zu atmen oder ist die Atemleistung ungenügend, werden die Organe nicht mehr ausreichend mit Sauerstoff versorgt. Eine künstliche Beatmung kann das Leben erleichtern oder sogar retten. Viele Situationen können eine künstliche Beatmung erfordern. Erkrankungen der Lunge zählen dazu, vor allem die chronisch obstruktive pneumonale Erkrankung (COPD) oder eine schwere Lungenentzündung. Infiziert sich die Lunge etwa an einem Virus wie SARS-CoV-2, verändert sich das Lungengewebe, Wasser tritt ein und der Sauerstoff erreicht aufgrund verringerter Diffusionskapazität die Blutbahn nicht mehr ausreichend. Steigt der Kohlenstoffdioxidgehalt, muss ein Patient beatmet werden. Auf der Intensivstation und im Operationssaal führen Beatmungsmaschinen die Beatmung durch. Das Beatmungsgerät beatmet die Lunge und erweitert aber auch den Brustkorb (Muskeln Knochen etc.). Eine Beatmungsmaschine ersetzt daher die Atmungsmuskulatur, nicht die Lunge. Die Aufgabe einer Beatmungsmaschine ist es, Kohlenstoffdioxid zu entfernen. Die Sauerstoffaufnahme geschieht dann nebenbei. Um das Kohlenstoffdioxid zu entfernen, atmen Gesunde ca 16 mal pro Minute etwa 0,5 Liter Luft bei Erkrankungen entsprechend mehr, z.B. wenn durch eine COVID19 Lungenentzündung weniger gesunde Lungen zur Entfernung von Kohlenstoffdioxid zur Verfügung steht. Dies leistet die Atemmuskulatur, die durch die Erkrankung sehr geschwächt und durch die hohe Last und durch Beatmungsschläuche sehr überlastet sein kann, so dass eine Atmung nicht oder kaum mehr möglich ist. Dann muss die Atmungsmuskulatur, wie bei einem Marathonläufer, Pausen einlegen, sich ausruhen und durch das Beatmungsgerät ersetzt werden. Eine geringere Last beim Mitatmen, ähnlich dem langsamer Gehen, reicht dabei häufig nicht aus. Die Muskeln ermüden doch. Beatmungsmaschinen erfordern meist eine Sedierung der Patienten, um den Atemreflex zu unterdrücken. Ein erheblicher Teil der COVID 19-Patienten überleben diese belastende Behandlung nicht. Bessere Behandlungschancen versprechen Beatmungsmaschinen, bei der Patienten nicht sediert werden müssen und bei denen der Patient nicht selbst atmen muss.

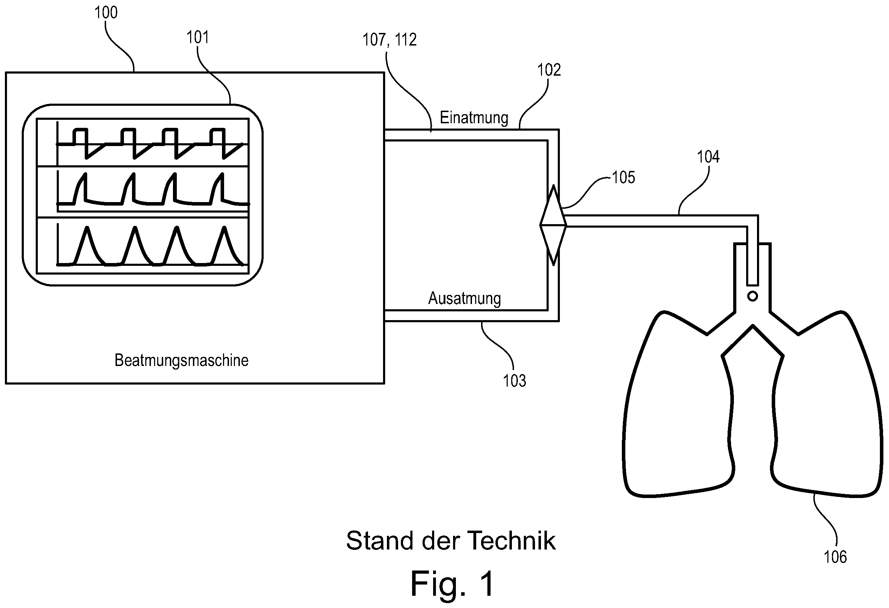

In einer vereinfachten Ausführung gemäß Figur 1 aus dem Stand der Technik weisen Beatmungsmaschinen 100 eine Überwachungseinheit 101 und eine Überdruckeinheit auf, wobei die Überdruckeinheit einen Atemgasstrom 107 bzw. einen Atemgas-Druckstrom 112 bereitstellt und den Lungen 106 des Patienten über einen Inspirationsschenkel 102 atembares Gas zuführt und über einen Exspirationsschenkel 103 ausgeatmete Luft wieder abführt. Inspirationsschenkel 102 und Exspirationsschenkel 103 sind über ein Patientenventil 105 mit einem gemeinsamen Inspirations- und Exspirationsschenkel 104 verbunden, welcher der Lunge 106 des Patienten invasiv oder nicht invasiv Luft zuführt. Je nachdem, ob der gemeinsame Inspirations- und Exspirationsschenkel 104 direkt in die Luftröhre geleitet wird oder mit Hilfe einer dicht schließenden Beatmungsmaske vom Patienten 106 über Mund und/oder Nase eingeatmet wird, wird allgemein von der invasiven oder nicht-invasiven Beatmung gesprochen.

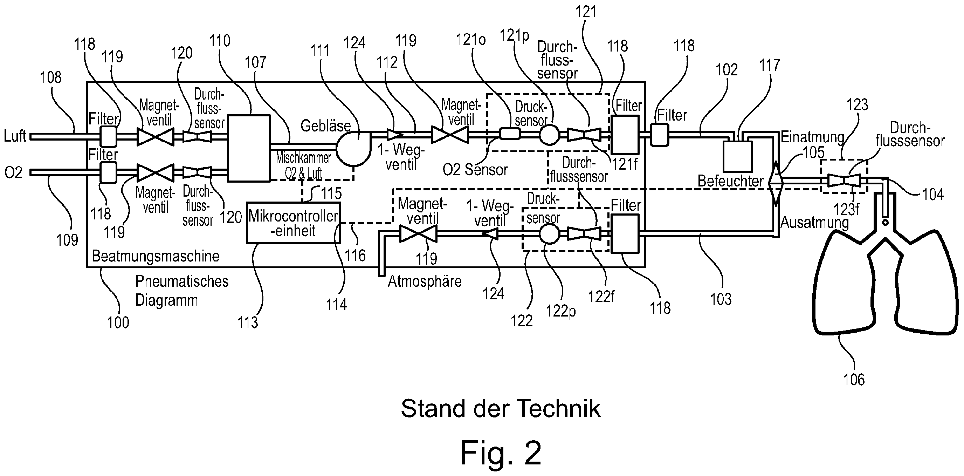

Figur 2 zeigt eine Beatmungsmaschine 100 aus dem Stand der Technik. Aus einem Ansaugstutzen 108 für Umgebungsluft wird Umgebungsluft und aus einem Ansaugstutzen 109 für Sauerstoff wird Sauerstoff in eine Mischeinheit 110 geleitet. Dabei wird in der Mischeinheit 110 ein Strom mit atembarer Luft 107 bereitgestellt. Eine Überdruckeinheit 111 beaufschlagt den Atemgasstrom 107 mit einem individuell einstellbaren Überdruck, bspw. 30 mbar zusätzlich gegenüber Umgebungsdruck, sodass ein Atemgas-Druckstrom 112 bereitsteht. Die Überdruckeinheit 111 ist hier als pulsierender Atemgasverdichter, bspw. Gebläse bzw. Turbine ausgeführt. Es wird beispielsweise eine Turbine benutzt, um den Patienten über ein Schlauchsystem mit Luft zu versorgen. Die Ein- und Ausatmung des Patienten wird dabei über eine spezifische Drehzahlregelung der Turbine entsprechend einer vorprogrammierten Prozedur erzeugt. Eine hohe Drehzahl erzeugt einen hohen Druck und bläst Luft in die Lunge. Bei kleiner Drehzahl fällt der Druck ab und der Patient kann ausatmen ("pulsierende" Beatmung).

Ein drehzahlgeregelter, elektrischer Antriebsmotor kann eine Drehzahl von bis zu 40.000 Umdrehungen pro Minute erreichen. Eine alternative Ausführung einer Überdruckeinheit 111 ist mit einem Druckluftreservoir realisierbar. Moderne Atemgasverdichter 111 sind durch eine Mikrocontrollereinheit (MCU) oder sonstige, elektronische Steuermittel 113 geregelt und erlauben eine Anpassung des Überdrucks bzw. Beatmungsdrucks des Atemgas-Druckstroms 112 und der Durchflusscharakteristik des Atemgas-Druckstroms 112. Magnetventile 119 und Einwegeventile 124 sind angeordnet, den Atemgasstrom 107 bzw. den Druckluftstrom 112 bei Bedarf zu unterbrechen bzw. in die gewünschten Richtungen zu leiten. Nach dem Befeuchten der Druckluft in einer Befeuchtungseinheit 117 wird der Atemgas-Druckstrom 112 über den gemeinsamen Inspirations- und Exspirationsschenkel 104 quasi in die Lunge 106 gedrückt. Wird der Atemgas-Druckstrom 112 gestoppt, atmet der Patient durch die Eigenelastizität der Lungen 106 passiv aus. Die ausgeatmete Luft wird durch den Exspirationsschenkel 103 abgeführt und an die Umgebung abgegeben. Sensoreinheiten 121, 122, 123 können erkennen, ob der Patient gerade einatmet oder ausatmet. Mittel zur Signalübertragung 116 geben den gemessenen Zustand an die MCU 113 weiter. Die Sensoreinheiten 121, 122, 123 können Durchflusssensoren121f,122f,123f, Drucksensoren 121p, 122p und/oder Sauerstoffsensoren 121o aufweisen. Messsignale der Sensoreinheiten 121, 122, 123 werden an die MCU 113 weitergegeben, woraufhin die MCU 113 ein entsprechendes Steuersignal an die Überdruckeinheit 111, die Mischeinheit 110 und/oder die Ventile 119 ausgeben kann.

Ferner können Beatmungsmaschinen 100 Monitore, Alarmsysteme für patientenbezogene Parameter (bspw. Druck, Volumen, Durchfluss) und gerätebezogene Funktionen (bspw. Leckagen, Stromversorgung, mechanische Störungen), Notstromversorgung, Sauerstofftanks und Fernbedienungseinrichtungen aufweisen.

Die im Stand der Technik gezeigte Vorrichtung bringt allerdings auch Nachteile mit sich. Die Atmung durch die Lungen 106 erkrankter Patienten ist typischerweise schnell und flach. Sie atmen bspw. 40 mal pro Minute, sodass ein Atemzug eine Dauer von 1,5 Sekunden aufweist. Erkennen die Sensoreinheiten 121, 122, 123, dass der Patient einatmet, verbleibt nur wenig Zeit, bspw. 400ms, damit durch Ansteuern der Überdruckeinheit 111 ausreichend Atemgas in Form eines Atemgas-Druckstroms 112 zur Verfügung steht.

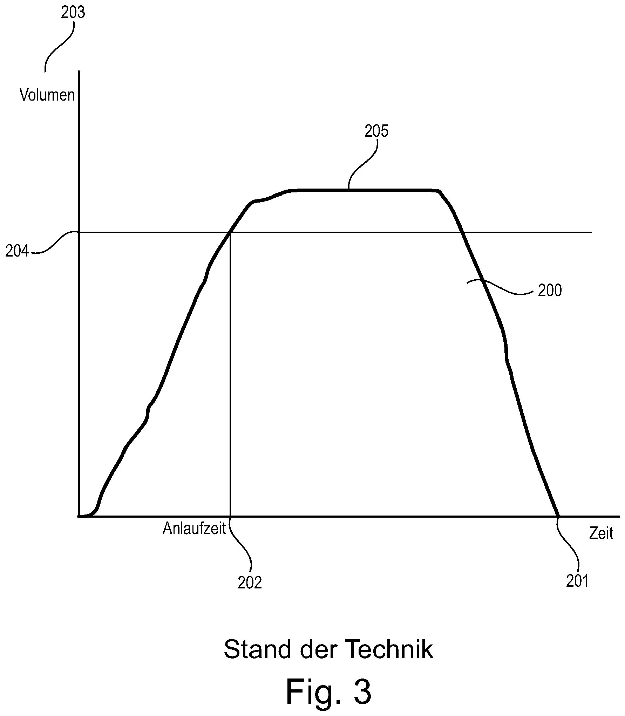

Bekannte Beatmungsmaschinen 100 stoßen an ihre Grenzen, wie anhand eines Diagramms gemäß Figur 3 veranschaulicht, das einen beispielhaften Atemgasvolumenstrom-Verlauf über die Zeit während der Applikation eines Tidalvolumens zeigt. Nach dem Erkennen des Einatmens benötigt die Beatmungsmaschine 100 Zeit, um ein ausreichendes Tidalvolumen 200 oder Atemzugvolumen während der Dauer 201, also bspw. 400-750 Millisekunden, des Einatmens zur Verfügung zu stellen. Das Tidalvolumen 200 entspricht allgemein dem Luftvolumen pro Atemzug und ist das eingestellte Volumen, bspw. 0,8 Liter Atemluft, das pro Atemhub appliziert werden soll. Die Anlaufzeit 202 resultiert aus zeitlichen Verzögerungen bei der Aktivierung der Elektronik, der Mechanik und des Luftstroms. Es vergeht bedingt durch die Systemträgheit eine Anlaufzeit 202, bis ein entsprechend der menschlichen Atmung pulsierender Atemgasverdichter 111 einen ausreichenden Atemgasvolumenstrom 203 zur Verfügung stellt, um einen voreingestellten Atemgasvolumenstrom (Inspirationsflow 204) zur erreichen. Der Inspirationsflow 204 ist der Wert für die Menge des in den Patienten einfließenden Gases bezogen auf die Zeit. Ein hoher Inspirationsflow 204 sorgt also für eine schnelle Belüftung der Lunge. Sobald der Patient mit dem Ausatmen beginnt, ist eine weitere Zufuhr von Atemgas-Druckluft 112 nicht mehr möglich. Der Atemgasvolumenstrom 203 wird durch entsprechende Ansteuerung des pulsierenden Atemgasverdichters 111 heruntergeregelt und schließlich gestoppt.

Im Mittel liegt der tatsächlich durch die Beatmungsmaschine 100 applizierte Inspirationsflow 205 immer unter dem eingestellten bzw. zur Applikation des eingestellten Tidalvolumens 200 erforderlichen Inspirationsflow 204. Gleichzeitig lässt sich der der Inspirationsflow 204 nicht beliebig erhöhen. So gilt ein Beatmungsdruck von 30 Millibar über Atmosphärendruck als Obergrenze für die Belastbarkeit der Lunge 106. Das erforderliche Tidalvolumen 200 kann so nicht erreicht werden. Der Behandlungserfolg ist durch die bekannten Lösungen damit durch technische Nachteile begrenzt.

Insbesondere Störungen der regelmäßigen Atmung durch Schlucken oder sonstige Irritationen sind für den beatmeten Patienten riskant. Damit Störungen vermieden werden können, müssen Beatmungspatienten in der Regel sediert werden.

Ferner sind aus der WO 99 / 08738 A1 bekannt eine Beatmungsmaschine zur Beatmung eines Patienten, wobei die Beatmungsmaschine mindestens eine Überdruckeinheit, insbesondere einen Atemgasverdichter, zum Bereitstellen eines Atemgas-Druckstroms in einem oder für einen Inspirationskanal und ein oder mehrere, die Überdruckeinheit kontrollierende, elektronische Steuerungsmittel (MCU) aufweist. Dabei ist das Steuerungsmittel (MCU) programm- und/oder schaltungstechnisch dazu eingerichtet, die Überdruckeinheit mit ihrem Atemgas-Druckstrom strömungstechnisch dem Inspirationskanal selektiv zu wenigstens einer vorab festgelegten Zeit- und/oder Triggerbedingung zuzuschalten und/oder von dem Inspirationskanal zu trennen.

In der US 5 485 850 A ist eine Beatmungsmaschine mit zwei verschiedenen Druckquellen zur Bereitstellung von Atemgasverdichtern aus verschiedenen Druckniveaus. Ein angelengtes Ventilbauteil schaltet an einem abzweigt entweder die erste Druckquelle oder die zeite Druckquelle zu.

Die US 2006 / 0 005 834 A1 betrifft einen Atemgasverdichter, der bei vergleichsweise konstanter Drehzahl läuft und durch ein der Atemgasstrom durch ein Durchflussventil geregelt ist.

In der US 2005 / 0 098 179 A1 sind zwei Atemgasverdichter angeordnet, um flexibler verschiedene Druckniveaus an Atemgas bereitstellen zu können. In der WO 00 21596 A1 sind ebenfalls zwei Atemgasverdichter in Reihe angeordnet.

Es ist die Aufgabe der vorliegenden Erfindung, die Nachteile aus dem Stand der Technik zu eliminieren und eine Beatmungsmaschine zur Verfügung zu stellen, mit der Patienten nicht sediert werden müssen. Ein weiteres bevorzugtes Ziel der Erfindung ist es, die Atemmuskulatur vollständig zu entlasten.

Die Aufgabe wird gelöst durch eine Beatmungsmaschine gemäß Anspruch 1. Vorteilhafte Ausführungsformen sind in den abhängigen Ansprüchen beansprucht und werden nachfolgend näher erläutert.

Die Erfindung betrifft eine Beatmungsmaschine zur Beatmung eines Patienten, wobei die Beatmungsmaschine eine Überdruckeinheit, insbesondere einen Atemgasverdichter, zum Bereitstellen eines Atemgas-Druckstroms in einem oder für einen Inspirationsschenkel aufweist. Dabei wird die Überdruckeinheit über entsprechend eingerichtete, elektronische Steuerungsmittel so angesteuert, dass die über Druckeinheit mit ihrem Atemgas-Druckstrom strömungstechnisch dem Inspirationskanal selektiv zu wenigstens einer vorab festgelegten Zeit- und/oder Triggerbedingung zuschaltbar und/oder von dem Inspirationskanal trennbar ist.

Gemäß einer optionalen, zweckmäßigen Erfindungsausbildung sind das oder die Steuerungsmittel programm- und/oder schaltungstechnisch dazu eingerichtet, die Überdruckeinheit als "konstante Überdruckeinheit" zum Bereitstellen konstanten Atemgas-Druckstroms oder eines Atemgas-Druckstroms, der zumindest konstant oder ständig in einem höheren Druckbereich liegt, anzusteuern oder zu betreiben, und der höhere Druckbereich einer Überdruck-Beatmung, Inspiration und/oder Hyper-Ventilation beim Patienten entspricht.

Gemäß einer optionalen Erfindungsausbildung stellt die konstante Überdruckeinheit während des Einatmens- und auch während des Ausatmens einen konstanten, d.h. einen im Wesentlichen unveränderten Atemgas-Druckstrom, d.h., bspw. mit einem konstanten Gasdruck von 30 Millibar Überdruck und einem konstanten Volumenstrom von 2 Litern pro Sekunde, in der Beatmungsmaschine zur Verfügung. Dabei ist der konstante Atemgas-Druckstrom während des Ausatmens durch ein Absperr- und/oder Mehrwegeventil und/oder eine sonstige Absperreinrichtung strömungstechnisch von dem beatmeten Patienten getrennt. Beim Einatmen wird die Trennung des Atemgas-Druckstroms bzw. der konstanten Überdruckeinheit zum Patienten aufgehoben, sodass dem Patienten über den Inspirationsschenkel Atemgas zugeführt werden kann. Das Einatmen durch den Patenten wird durch eine vorab festgelegte Zeitbedingung, bspw. alle 1,5 Sekunden, oder eine vorab festgelegte Triggerbedingung, bspw. das Messsignal einer Sensoreinheit, erkannt. Die Anlaufzeit, die eine gepulste Überdruckeinheit typischerweise benötigt, um einen voreingestellten Inspirationsflow zu erreichen, kann durch den durchlaufenden und konstanten Betrieb der Überdruckeinheit erheblich verkürzt werden oder sogar vollständig entfallen.

Durch die Erfindung kann der Behandlungserfolg, bspw. bei einer COVID19-Lungenentzündung, verbessert werden. Bei der Behandlung von COVID19 besteht die Ansicht, dass durch die Beatmung Lungenschäden eintreten, Wasser in die Lunge eintritt und die Atmung erschwert. Es hat sich jedoch gezeigt, dass diese Ansicht zumindest bei einem Großteil der Patienten nicht zutrifft und z.B. COVID19 Patienten ihre Erkrankung überleben, wenn ihre Atmungsmuskulatur für die Abgabe von Kohlenstoffdioxid vollständig entlastet wird. Beatmungsgeräte die eine vollständige Entlastung der Atmungsmuskulatur automatisiert sicher stellen, können daher den Behandlungserfolg steigern. Solche Beatmungsmaschinen sind im Stand der Technik derzeit unbekannt. Durch die passive Beatmung der Erfindung kann der Patient nicht nur entlastet, sondern vollständig entlastet und überbeatmet werden. Der Patient verspürt allein durch das Beatmungsgerät dauerhaft keinen Atemantrieb mehr, ohne dass der Atemantrieb durch Medikamente unterdrückt werden muss.

In einer vorteilhaften Weiterbildung der Erfindung ist die Triggerbedingung durch ein oder mehrere Messignale aus einer oder mehreren Sensoreinheiten feststellbar.

Bei der Beatmung werden im Stand der Technik als Reservegeräte Beatmungsbeutel eingesetzt, mit denen der beatmete Patient bei Atemnot durch manuelle Betätigung des Beatmungsbeutels hyperventiliert werden kann. Der Beatmungsbeutel ist allgemein ein Hilfsmittel zur manuellen Beatmung von Patienten mit Atemstillstand oder insuffizienter (nicht ausreichender) Atmung. Hyperventilieren heißt allgemein, dass dem Patienten mehr Luft zur Verfügung gestellt wird, als der Patient braucht. Mit dem Beatmungsbeutel kann ein leichter Überdruck appliziert werden. Durch Betätigung des Beatmungsbeutels wird die Atemnot des Patienten behoben und der ist nicht mehr geneigt, gegen das Gerät zu atmen. Bekannt sind im Stand der Technik ferner Maschinen zur "pulsierenden" Atemunterstützung, die nach einem festen und vorab festgelegten Muster oder Rhythmus, z.B. bei jedem Atemzug und/oder bei jeweils drei Atemzügen in Folge hyperventilieren und somit ausschließlich in Abhängigkeit des vorab vorprogrammierten Ablaufs Atemluft applizieren. Wenn der Patient von einer Beatmungsmaschine beatmet wird, wird permanent ein Überdruck appliziert. Atmet der Patient jedoch bei einem Atemzug spontan mit, so fällt der Beatmungsdruck sofort ab, es ist mit zusätzlichem Atemgasvolumen gegenzusteuern. Dieser Vorgang ist daher vorzugsweise zu automatisieren. Die Feststellung der Triggerbedingung durch die Anordnung von Sensoreinheiten zum Erfassen von geeigneten Messwerten, kann das Applizieren zusätzlicher Atemluft automatisieren. Dies kann die Zuverlässigkeit der Beatmungsmaschine verbessern und die Reaktionszeit bis zur Applikation des Hyperventilierens verkürzen. Auch eine Veränderung des Atemrhythmus kann durch Sensoreinheiten schneller festgestellt werden, als durch menschliche Beobachtung. Durch die Festlegung eindeutiger Triggerbedingungen mit Hilfe von Sensoreinheiten kann die Eignung als Notfallsystem verbessert werden. Zur Automatisierung ist eine Steuer-/Regelungs-/Speicher- und/oder Recheneinheit, insbesondere ein Microcontroller (MCU) vorgesehen, wobei die Steuer-/Regelungs-/Speicher- und/oder Recheneinheit ein Stellsignal zum Bereitstellen des konstanten Atemgas-Druckstroms ausgibt. Dabei wird das Stellsignal als Reaktion auf ein Messignal einer oder mehrerer Sensoreinheiten ermittelt. Durch die Messsignale kann festgestellt werden, ob der Patient einatmet bzw. versucht, einzuatmen. Ein Vorteil besteht in der dauerhaften Verhinderung der Eigenatmung durch sofortige Intervention.

Vorzugsweise ist mindestens eine Sensoreinheit zum Feststellen der Triggerbedingung in einem gemeinsamen bzw. gleichzeitigen Inspirations- und Exspirationsschenkel der Beatmungsmaschine angeordnet.

Durch die Anordnung mit einem möglichst geringem räumlichen Abstand zum Patienten wird der zeitliche Abstand zwischen dem tatsächlichen Einatmen und dem Erkennen des Einatmens durch eine Sensoreinheit, bspw. durch Erkennen eines Druckabfalls im Atemgas-Druckstrom, verkürzt. Das Messignal, welches beim Einatmen generiert wird, steht schneller zur Verfügung und die Anlaufzeit für die vorgesehene Reaktion durch Ventile und/oder Überdruckeinheiten kann verkürzt werden. Der Zustand der Atemnot wird für den Patienten verkürzt, was den Behandlungserfolg durch die Beatmungsmaschine steigert.

In einer optionalen Weiterbildung weisen die einen oder mehreren Sensoreinheiten zum Feststellen der Triggerbedingung einen Drucksensor zur quantitativen Erfassung des Atemgasdrucks und/oder einen Durchflusssensor zur quantitativen Erfassung des Atemgasdruckstroms und/oder einen Sauerstoffsensor zur quantitativen Erfassung des Sauerstoffgehalts im Atemgas auf.

Durch die Anordnung verschiedener Sensoren an verschiedenen Stellen in der Beatmungsmaschine stehen mehrere Messsignale und insbesondere redundante Messsignale zur Verfügung, um den Bedarf einer Applikation eines zusätzlichen Atemgas-Druckstroms zuverlässig und zeitnah zu ermitteln und den Betrieb der Überdruckeinheiten und/oder der Mischeinheit entsprechend zu justieren. Die Sensorelemente können auch einen Differenzdruckmesser aufweisen, die einen Druckabfall sensieren und so einen Atemreflex durch Spontanatmung oder Eigenatmung des Patienten erkennen.

In einer bevorzugten Ausführung ist die konstante Überdruckeinheit als Atemgasverdichter, bspw. Rotationsgebläse oder Hubkolbenverdichter, ausgebildet, der druckseitig oder ausgangsseitig einen konstanten oder konstant in einem bestimmten höheren Druckbereich liegenden, vorab festlegbaren Überdruck oder Beatmungsdruck bereitstellen kann.

Der Atemgasverdichter ist vorzugsweise als Gebläse ausgebildet, das während der Beatmung des Patienten bei einer konstanten Drehzahl, bspw. bei 40.000 Umdrehungen pro Minute eingangsseitig einen Atemgasstrom ansaugt und ausgangsseitig oder druckseitig einen Atemgas-Druckstrom ausgibt. Der Atemgas-Druckstrom wird damit konstant oder kontinuierlich in einem höheren Druckbereich liegend und auf Vorrat bereitgestellt, ohne dass ein Reservoir, bspw. in Form einer Gasflasche bereitstehen muss. Eine Reduktion der Umdrehungszahl während des Ausatmens findet bevorzugt nicht statt, vielmehr lässt man den Atemgas-Druckstrom während des Ausatmens vorzugsweise in der Beatmungsmaschine intern zirkulieren, und/der er wird ungenutzt an die Umgebung abgegeben. Es können dem Patienten allein durch Veränderung von Ventilstellungen sofort hohe Volumenströme an Atemluft bei gleichzeitig maximal zulässigem Beatmungsdruck zur Verfügung gestellt werden. Der Patient wird hyperventiliert. Der Patient kann durch die konstante Überdruckeinheit bei jedem Atemzug vorsorglich passiv hyperventiliert werden.

Grundsätzlich sind Atemgasverdichter mit linear arbeitenden oder rotatorisch arbeitenden Antriebseinheiten einsetzbar.

Vorzugsweise ist der Atemgasverdichter zum Ansaugen von Atemgas eingangsseitig oder saugseitig mit einer Mischeinheit verbunden oder verbindbar. In der Mischeinheit kann einem angesaugten Umgebungsluftstrom nach Bedarf zusätzlicher Sauerstoff zugemischt werden.

Ferner kann vorgesehen sein, dass der Atemgasverdichter zum Ansaugen von Umgebungsluft eingangsseitig oder saugseitig mit der Umgebung und/oder über eine Bypassleitung zum Umgehen der Mischeinheit mit einem Ansaugstutzen der Beatmungsmaschine und/oder der Mischkammer verbunden oder verbindbar ist.

Durch eine entsprechende Anordnung von einem Mehrwegeventil oder vergleichbaren Apparaturen saugseitig des Atemgasverdichters, kann während der Trennung des Atemgas-Druckstroms vom Patienten die Ventilstellung so gewählt werden, dass Umgebungsluft anstelle von mit Sauerstoff angereichertem Atemgas ansaugbar ist. Dadurch kann eine Einsparung von reinem Sauerstoff zum Anmischen des Atemgasstroms erreicht werden.

Zusätzlich zu der konstanten Überdruckeinheit zum Bereitstellen eines konstanten oder konstant/kontinuierlich in einem höheren Druckbereich liegenden Atemgas-Druckstroms ist eine weitere, "pulsierende" (siehe oben) Überdruckeinheit zum Bereitstellen eines pulsierenden Atemgas-Druckstroms angeordnet.

Die pulsierende Überdruckeinheit startet mit Beginn des Einatmens und stellt mit einer zeitlichen Verzögerung, d.h. mit einer Anlaufzeit einen Atemgas-Druckstrom mit einem vorab festgelegten maximalen Gasdruck von bspw. 30 Millibar Überdruck zum Applizieren eines vorab festgelegten Tidalvolumens zur Verfügung. Durch die Verwendung einer pulsierenden Überdruckeinheit können Absperrventile zum Trennen dieser (pulsierenden) Überdruckeinheit von einem zum Patienten führenden Schlauchsystem während des Ausatmens entfallen. Durch die Anordnung einer zweiten, insbesondere konstanten Überdruckeinheit ist eine redundante Atemluft-Versorgung des Patienten gewährleistet. Anstelle oder zusätzlich zu einer gepulsten Überdruckeinheit sind auch eine oder mehrere konstante Überdruckeinheiten einsetzbar. Auch für die pulsierende Überdruckeinheit sind alle bekannten linearen oder rotatorischen Antriebseinheiten, bspw. Gebläse oder Kolbenpumpen, verwendbar. Die Überdruckeinheiten sind vorzugsweise unabhängig voneinander ansteuerbar. Ferner ist der durch die konstante Überdruckeinheit erzeugte Atemgas-Druckstrom von dem Atemgas-Druckstrom der pulsierenden Überdruckeinheit unabhängig erzeugbar. In anderen Worten, die konstante Überdruckeinheit läuft unabhängig und entkoppelt von der pulsierenden Überdruckeinheit und ist auf Anforderung, also bspw. durch den Eintritt einer Triggerbedingung, wie dem Einatmen, durch ein Ventilsystem mittels des oder der elektronischen Steuerungsmittel zuschaltbar. Wenn bspw. die pulsierende Überdruckeinheit einen vorab festgelegten Beatmungsdruck überschreitet, wird mittels der Steuerungsmittel die gepulste Überdruckeinheit quasi abgeschaltet, oder sie wird vom Patienten getrennt und die konstante Überdruckeinheit ersetzt auf ihre spezifische Weise die Beatmung durch die gepulste Überdruckeinheit. Die Zuschaltung erfolgt selektiv. Das oder die Steuerungsmittel ergeben hierfür ein Steuersignal an das Ventilsystem zum Zuschalten des zweiten Atemgas-Druckstroms aus.

In einer bevorzugten Weiterbildung ist die konstante Überdruckeinheit zum Bereitstellen eines gepulsten Atemgas-Druckstroms und die gepulste Überdruckeinheit zum Bereitstellen eines konstanten Atemgas-Druckstroms oder eines Atemgas-Druckstroms, der zumindest konstant oder ständig in einem höheren Druckbereich liegt, ausgebildet. In anderen Worten, die Drehzahl des konstanten Atemgasverdichters könnte bei Bedarf pulsierend reduziert werden, während die Drehzahl des gepulsten Atemgasverdichters bei Bedarf konstant oder konstant in einem höheren Druckbereich gehalten werden kann. Durch diese Redundanz-Maßnahmen werden die Zuverlässigkeit und Ausfallsicherheit der Beatmungsmaschine weiter verbessert.

In einer möglichen Ausführungsform sind die pulsierende Überdruckeinheit und die konstante Überdruckeinheit strömungstechnisch in Reihe angeordnet oder anordenbar, wobei vorzugsweise die konstante Überdruckeinheit eingangsseitig oder saugseitig der pulsierenden Überdruckeinheit angeordnet oder anordenbar ist. In anderen Worten, zunächst erzeugt die konstante Überdruckeinheit einen Atemgas-Druckstrom mit einem Beatmungsdruck von bspw. 15 Millibar über Atmosphärendruck, der anschließend von der pulsierenden Überdruckeinheit weiter auf 30 Millibar erhöht werden kann. Durch die Vorverdichtung kann die pulsierende Überdruckeinheit einen vorgegebenen Beatmungsdruck und einen vorgegebenen Inspirationsflow schneller erreichen.

Alternativ und/oder zusätzlich zu einer seriellen Anordnung können die pulsierende Überdruckeinheit und die konstante Überdruckeinheit strömungstechnisch parallel angeordnet oder anordenbar sein, wobei sich ein der pulsierenden Überdruckeinheit zugeordneter Teilstrom des Atemgas-Druckstroms und ein der konstanten Überdruckeinheit zugeordneter zweiter Teilstrom des Atemgas-Druckstroms druckseitig oder ausgangsseitig im Inspirationsschenkel oder im gemeinsamen Inspirations- und Exspirationsschenkel der Beatmungsmaschine vereinigen.

Durch die parallele Anordnung sind die Überdruckeinheiten mit eigenen Strömungspfaden unabhängig voneinander zu einer Beatmung des Patienten ausgebildet, was die Redundanz und damit Ausfallsicherheit und Flexibilität verbessert. Es können dadurch höhere Volumenströme an einem Atemluft-Druckstrom bereitgestellt werden. Ob die Überdruckeinheiten der Beatmungsmaschine parallel oder seriell betrieben werden, entscheidet ein Algorithmus, der auf der oder den elektronischen Steuerungsmittel hinterlegt sein kann

In einer bevorzugten Ausführungsform der Erfindung weist die Beatmungsmaschine zwei Betriebsmodi auf, wobei in einem ersten Betriebsmodus die Beatmungsmaschine zum Bereitstellen eines pulsierenden Atemgas-Druckstroms durch die pulsierende Überdruckeinheit ausgebildet ist. In einem zweiten Betriebsmodus ist die Beatmungsmaschine zum Bereitstellen eines konstanten Atemgas-Druckstroms oder eines Atemgas-Druckstroms, der zumindest konstant oder ständig in einem höheren Druckbereich liegt, durch die konstante Überdruckeinheit ausgebildet.

Die pulsierende Überdruckeinheit übernimmt die regelmäßige Beatmung während des ersten Betriebsmodus. Der Patient verspürt ein Gefühl, als hätte er gerade gegebenenfalls tief Luft geholt, um bspw. zu tauchen. Der Patient verspürt keine Atemnot und keinen Drang zum aktiven Einatmen. Die konstante Überdruckeinheit ist währenddessen strömungstechnisch isoliert von der ersten Überdruckeinheit angeordnet. Wird der erste Betriebsmodus gestört durch eine Schluckbewegung oder eine Irritation des Patienten, kann er Atemnot verspüren und nach Luft schnappen.

Dieser Fall wird durch ein Messsignal einer Sensoreinheit ermittelt, was als Triggerbedingung den zweiten Betriebsmodus während des gleichen Atemzugs einleitet, wobei der zweite Betriebsmodus den Patienten passiv hyperventiliert. Dabei wird der durch die konstante Überdruckeinheit erzeugte, redundante oder ergänzende Atemgas-Druckstrom über Ventile selektiv zugeschaltet und vorzugsweise mit dem ersten Atemgas-Druckstrom zu einem Hauptluftstrom im Inspirationsschenkel oder im gemeinsamen Inspirations- und Exspirationsschenkel vereinigt. In anderen Worten, wenn durch Eigeneinatmung oder Spontanatmung des Patienten, Luft angesaugt wird (anstatt durch das Beatmungsgerät hineingeblasen) und der Beatmungsdruck in der Beatmungsmaschine damit abfällt, wird die Überdruckeinheit sofort für den gleichen Atemzug zugeschaltet. Dadurch stellt die Beatmungsmaschine für den nächsten Atemzug wieder eine Überbeatmungssituation her, so dass der Patient nicht mehr selbst einatmen muss oder will. Die Atemmuskulatur bleibt entlastet.

Vorzugsweise ist in dem zweiten Betriebsmodus die Beatmungsmaschine zum Bereitstellen eines pulsierenden Atemgas-Druckstroms durch die pulsierende Überdruckeinheit ausgebildet. In anderen Worten, während des zweiten Betriebsmodus können sich die konstante Überdruckeinheit und die gepulste Überdruckeinheit gegenseitig ergänzen. Der zweite Betriebsmodus kann grundsätzlich auch dann gestartet werden, wenn irgendeine Störung des ersten Betriebsmodus, bspw. ein Ausfall der pulsierenden Überdruckeinheit, eintritt. Muss bspw. die pulsierende Überdruckeinheit vom Patienten getrennt werden, damit kein zu hoher Beatmungsdruck auf den Patienten appliziert wird, steht sofort die konstante Überdruckeinheit in Reserve und kann durch die Steuerungsmittel über ein Ventil zugeschaltet werden, damit ein Atemgas-Druckstrom sofort zum Beatmen des Patienten zur Verfügung steht. Es liegt quasi ein redundantes System vor. Der Patient kann ununterbrochen beatmet werden.

In einer bevorzugten Weiterbildung kann in dem zweiten Betriebsmodus die konstante Überdruckeinheit einen konstanten Atemgas-Druckstroms bereitstellen, bis der pulsierende Atemgas-Druckstrom durch die pulsierende Überdruckeinheit einen vorab festgelegten Atemgasvolumenstrom, also den Inspirationsflow, und/oder einen vorab festgelegten Gasdruck erreicht.

Wenn der Patient während des ersten Betriebsmodus versucht, wegen Atemnot vorzeitig einzuatmen und die pulsierende Überdruckeinheit die Atemnot wegen der unvermeidbaren Anlaufzeit nicht rechtzeitig lindern kann, wird beispielsweise mittels der elektronischen Steuerungsmittel der zweite Betriebsmodus aktiviert. Im zweiten Betriebsmodus wird die Anlaufzeit der pulsierenden Überdruckeinheit durch eine Ventilöffnung und ein Hinzuschalten der konstanten Überdruckeinheit überbrückt, sodass sofort ausreichend Atem lauft bereitsteht. Sobald die pulsierende Überdruckeinheit ausreichend Atemluft bereitstellt, kann die konstante Überdruckeinheit wieder vom Patienten getrennt werden. Dabei geben die Steuerungsmittel ein Steuersignal an das betroffene Ventil zum Zuschalten des zweiten Atemgas-Druckstroms aus. Es können dem Patienten sofort hohe Volumenströme an Atemluft bei gleichzeitig maximal zulässigem Beatmungsdruck zur Verfügung gestellt werden. Der Patient wird passiv hyperventiliert. Durch die Zuschaltung eines zweiten Atemgas-Druckstroms kann selbst bei schneller Atmung dem Patienten innerhalb eines entsprechend verkürzten Zeitfensters ein ausreichendes Tidalvolumen zur Verfügung gestellt werden. Bereits ein Einatmen von 400 Millisekunden oder weniger kann dann ausreichen, um den Patienten mit ausreichend Atemgas zu versorgen. Die Patienten müssen nicht mehr sediert werden, was den Behandlungserfolg für die Patienten insgesamt verbessert. Bei unterdessen ausreichendem Atemgasvolumenstrom durch die pulsierende Überdruckeinheit kann die konstante Überdruckeinheit wieder vom Patienten getrennt werden.

Weitere Einzelheiten, Merkmale, Merkmals(unter) kombinationen, Vorteile und Wirkungen auf der Basis der Erfindung ergeben sich aus der nachfolgenden Beschreibung eines bevorzugten Ausführungsformen der Erfindung und den Zeichnungen. Diese zeigen in
- Fig. 1: ein vereinfachtes Blockschaltbild einer Beatmungsmaschine aus dem Stand der Technik, in
- Fig. 2: ein Verfahrensfließbild einer Beatmungsmaschine aus dem Stand der Technik, in
- Fig. 3: ein Diagramm, darstellend einen beispielhaften Atemgasvolumenstrom-Verlauf während der Applikation eines Tidalvolumens über die Zeit aus dem Stand der Technik, in
- Fig. 4: ein Verfahrensfließbild einer Beatmungsmaschine in einer beispielhaften Ausführungsform der Erfindung, in
- Fig. 5a: ein Verfahrensfließbild einer Beatmungsmaschine mit einem ersten beispielhaften Betriebsmodus in einer beispielhaften Ausführungsform der Erfindung, in
- Fig. 5b: ein Verfahrensfließbild einer Beatmungsmaschine mit einem zweiten Betriebsmodus in einer beispielhaften Ausführungsform der Erfindung, in
- Fig. 5c: ein Verfahrensfließbild einer Beatmungsmaschine mit einem weiteren zweiten Betriebsmodus in einer beispielhaften Ausführungsform der Erfindung und in
- Fig. 5d: ein Verfahrensfließbild einer Beatmungsmaschine mit einem weiteren zweiten Betriebsmodus in einer beispielhaften Ausführungsform der Erfindung.

Die Figuren sind lediglich beispielhafter Natur und dienen nur dem Verständnis der Erfindung. Die gleichen Elemente sind mit denselben Bezugszeichen versehen. Zur Beschreibung bereits bekannter Komponenten aus dem Stand der Technik wird auf die Figurenbeschreibung zu den Figuren 1 - 3 in der Beschreibungseinleitung verwiesen.

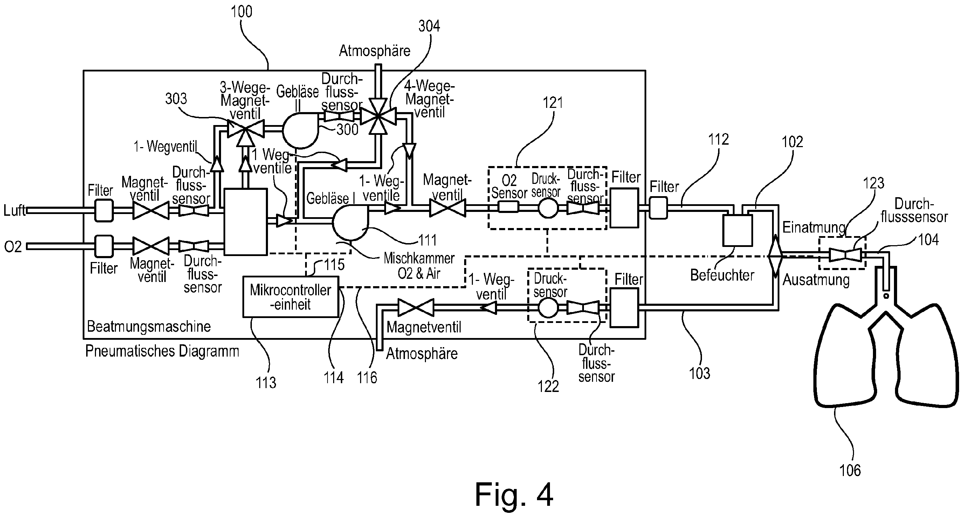

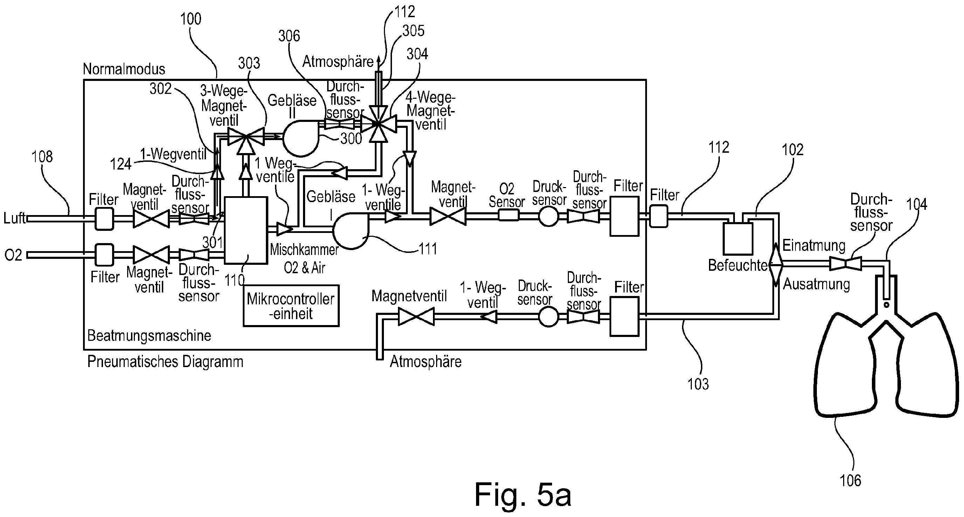

Fig. 4 zeigt eine erste Ausführungsform einer erfindungsgemäßen Beatmungsmaschine 100. Die Beatmungsmaschine 100 weist zusätzlich zu einem pulsierend betriebenen Atemgasverdichter 111 einen konstant oder ständig in einem höheren Druckbereich betriebenen Atemgasverdichter 300 auf. Die Atemgas-Druckströme 112 können sich in einem Inspirationsschenkel 102 vereinigen. Durch eine Sensoreinheit 121 im Inspirationsschenkel 102, eine Sensoreinheit 122 im Exspirationsschenkel 103 und/oder eine Sensoreinheit 123 im gemeinsamen Inspirations- und Exspirationsschenkel 104 können Messsignale generiert werden, die über (gestrichelt angedeutete) Signalleitungen 116 einer oder mehrerer elektronischer Steuerungsmittel 113, im Beispiel dem Mikrocontroller MCU, übermittelbar sind. Der Mikrocontroller MCU 113 empfängt die Messsignale an einem oder mehreren Messsignaleingängen 114 und gibt anhand vorab festgelegter Triggerbedingungen an Steuersignalausgängen 115 Steuersignale an bspw. die Atemgasverdichter 111, 300 und/oder die Mischeinheit 110 und/oder die Mehrwegeventile (vgl. Fig. 5a-5d) aus. Je nach Vorgabe, kann der Patient 106 durch den einen Atemgasverdichter und/oder durch den anderen Atemgasverdichter 111, 300 beatmet werden.

Figur 5a zeigt die Beatmungsmaschine 100 während eines ersten Betriebsmodus. Der pulsierende Atemgasverdichter 111 versorgt den Patienten 106 mit einem Atemgas-Druckstrom 112, der nach dem Ansaugen aus der Mischeinheit 110 auf ca. 30 Millibar verdichtet worden ist. Währenddessen ist der konstante Atemgasverdichter 300 quasi in Bereitschaft, um bei Bedarf ohne Anlaufzeit 202 (vgl. Figur 3) den Patienten 106 hyperventilieren zu können. Hierfür entnimmt der konstante Atemgasverdichter 300 über eine Bypassleitung 302 an einem Abzweig 301 Umgebungsluft aus dem Ansaugstutzen 108 für Umgebungsluft, bevor die Umgebungsluft in die Mischeinheit 110 gelangt. Ein Einwegeventil 124 in der Bypassleitung 302 verhindert ein Rückströmen der angesaugten Umgebungsluft. Ein Dreiwegeventil 303 in zwischen der Bypassleitung 302 und dem Ansaugstutzen des konstanten Atemgasverdichters 300 befindet sich für ein Ansaugen von Umgebungsluft in einer ersten Ventilstellung. Da im ersten Betriebsmodus während der Bereitschaft des konstanten Atemgasverdichters 300 die durch ihn erzeugte Druckluft 112 nicht benötigt wird, wird sie durch einen Ausblasstutzen 305 wieder an die Umgebung abgegeben. Hierfür befindet sich ein Vierwegeventil 304 an oder nach dem Druckstutzen des konstanten Atemgasverdichters 300 in einer ersten Ventilstellung. Über einen Durchflusssensor 306 ist der Volumenstrom zwischen dem konstanten Atemgasverdichter 300 und dem Vierwegeventil 304 bestimmbar. Durch den Bereitschaftsmodus des konstanten Atemgasverdichters 300 während des ersten Betriebsmodus der Beatmungsmaschine 100 kann Sauerstoff eingespart werden.

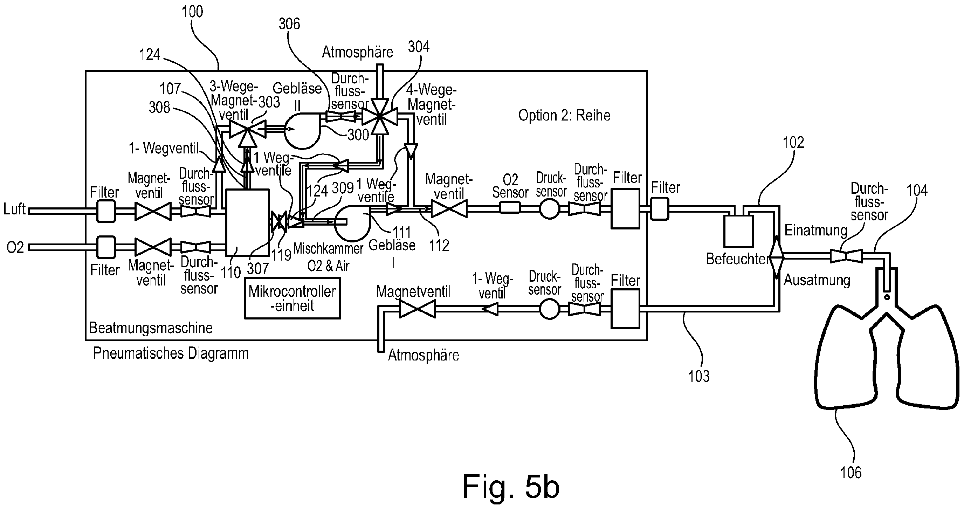

Figur 5b zeigt die Beatmungsmaschine 100 während eines zweiten Betriebsmodus. Der pulsierende Atemgasverdichter 111 versorgt den Patienten 106 mit einem Atemgas-Druckstrom 112, wobei saugseitig des gepulsten Atemgasverdichters 111 mittels des entsprechend elektronisch angesteuerten Vierwegeventils 304 der konstante Atemgasverdichter 300 mit seinem Ausgang angeordnet ist. Der konstante Atemgasverdichter 300 saugt einen Atemgasstrom 107 direkt aus der Mischeinheit 110 an. Die Bypassleitung 302 wird nicht mehr durchströmt, das Dreiwegeventil 303 zwischen der Bypassleitung 302 und dem Ansaugstutzen des konstanten Atemgasverdichters 300 befindet sich für ein Ansaugen eines Atemgasstroms 107 aus der Mischeinheit 110 in einer entsprechenden, zweiten Ventilstellung. Ein Einwegeventil 124 zwischen einem Mischeinheitausgang 308 und dem Dreiwegeventil 303 verhindert ein Rückströmen des angesaugten Atemgases in die Mischeinheit 110. Der im konstanten Atemgasverdichter 300 erzeugte Atemgas-Druckstrom wird für eine serielle Anordnung der beiden Atemgasverdichter 111, 300 an einer der seriellen Anordnung zugeordneten Einmündung 309 in einen Leitungsabschnitt zwischen einem Mischeinheitausgang 307 und dem Ansaugeingang des pulsierenden Atemgasverdichters 111 geführt. Hierfür befindet sich das Vierwegeventil 304 an oder nach dem Druckstutzen des konstanten Atemgasverdichters 300 in einer entsprechenden, zweiten Ventilstellung. Indem der konstante Atemgasverdichter 300 mit seinem Ausgang in den Bereich des Saugstutzens des pulsierenden Atemgasverdichters 111 einwirkt, lässt sich in Verbindung mit dem pulsierenden Atemgasverdichter 111 beim Einatmen des Patienten wesentlich schneller ein voreingestellter Inspirationsflow 204 erreichen. Die Anlaufzeit 202 (vgl. Figur 3) kann damit verkürzt oder im besten Fall vollständig überbrückt werden. Ein Einwegeventil 124 und ein Magnetventil 119 in einem Leitungsabschnitt zwischen der seriellen Anordnung zugeordneten Einmündung 309 und dem Mischeinheitausgang 307 verhindern ein Rückströmen des durch den konstanten Atemgasverdichters 300 erzeugten Atemgas-Druckstroms 112 und ein Ansaugen des pulsierenden Atemgasverdichters 111 aus der Mischeinheit 110.

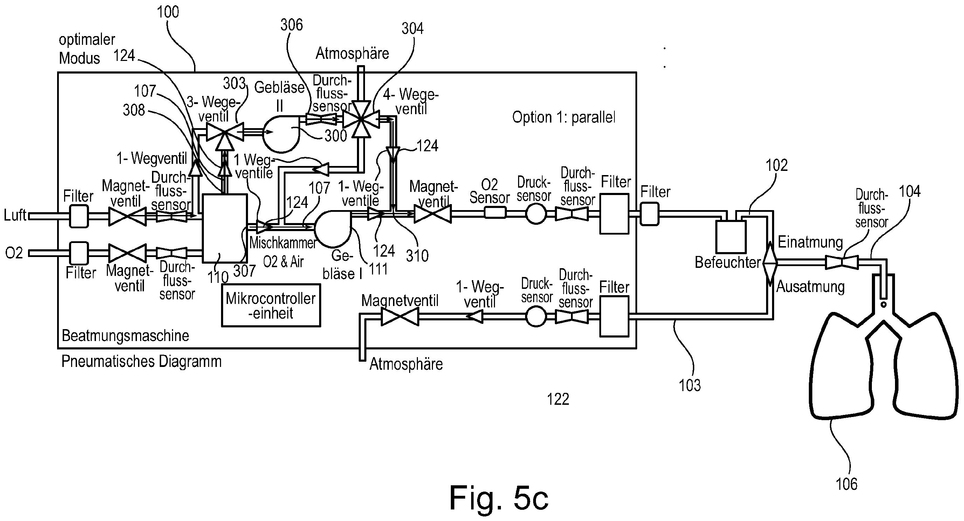

Figur 5c zeigt die Beatmungsmaschine 100 während eines weiteren zweiten Betriebsmodus. Der pulsierende Atemgasverdichter 111 und der konstante Atemgasverdichter 300 versorgen den Patienten 106 mit einem Atemgas-Druckstrom 112, wobei jeweils saugseitig des pulsierenden Atemgasverdichters 111 und des konstanten Atemgasverdichters 300 die Mischeinheit 110 angeordnet ist, ein Atemgasstrom 107 also direkt aus der Mischeinheit 110 angesaugt wird. Das Dreiwegeventil 303 zwischen der Mischeinheit 110 und dem Saugstutzen des konstanten Atemgasverdichters 300 befindet sich für ein Ansaugen von einem Atemgasstrom 107 in der entsprechenden, zweiten Ventilstellung. Ein Einwegeventil 124 zwischen einem Mischeinheitausgang 308 und dem Dreiwegeventil 302 verhindert ein Rückströmen des angesaugten Atemgases in die Mischeinheit 110. Der im konstanten Atemgasverdichters 300 erzeugte Atemgas-Druckstrom wird für eine parallele Anordnung der Atemgasverdichter 111, 300 an einer der parallelen Anordnung zugeordneten Einmündung 310 in einen Leitungsabschnitt zwischen dem Druckstutzen des pulsierenden Atemgasverdichters 111 und dem gemeinsamen, direkt zu den Lungen 106 des Patienten führenden Inspirations- und Exspirationsschenkel 104 geführt. Hierfür befindet sich das Vierwegeventil 304 an oder nach dem Druckstutzen des konstanten Atemgasverdichters 300 in einer entsprechenden, dritten Ventilstellung. Der pulsierende Atemgasverdichter 111 wird dabei unabhängig von dem konstanten Atemgasverdichter 300 weiterbetrieben, sodass dem Patienten 106 mittels zwei Teilströmen ein größeres Volumen Atemgas-Druckstrom 112 zur Verfügung gestellt werden kann. Durch den bereits vorab im höheren Druckbereich laufenden, konstanten Atemgasverdichter 300 kann eine Anlaufzeit des pulsierenden Atemgasverdichters 111 überbrückt werden, bis der pulsierende Atemgasverdichter 111 beim Einatmen einen voreingestellten Inspirationsflow 204 erreicht hat. Die Anlaufzeit 202 (vgl. Figur 3) kann damit verkürzt oder im besten Fall vollständig überbrückt werden.

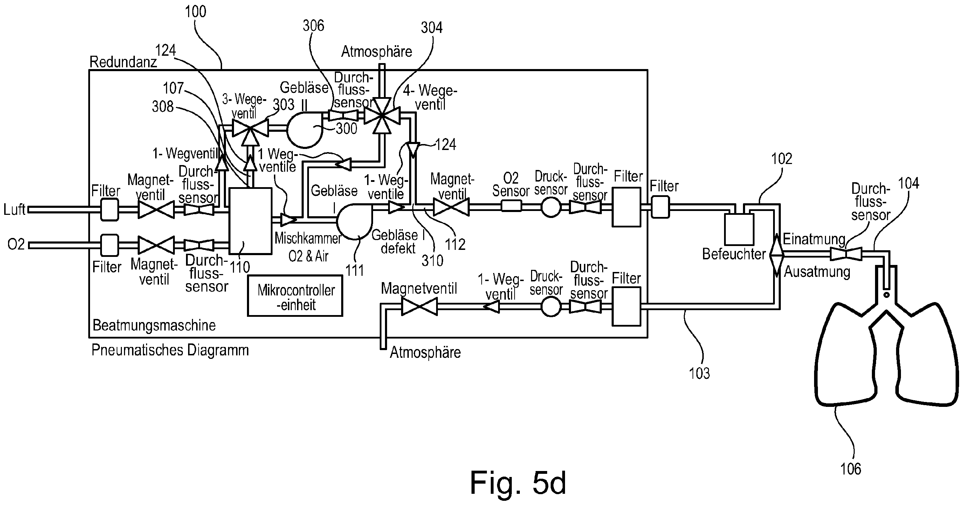

Figur 5d zeigt die Beatmungsmaschine 100 während eines weiteren zweiten Betriebsmodus. Der konstante Atemgasverdichter 300 versorgt die Patientenlunge 106 allein mit einem Atemgas-Druckstrom 112, wobei saugseitig des konstanten Atemgasverdichters 300 die Mischeinheit 110 angeordnet ist. Ein Teilstrom aus dem pulsierenden Atemgasverdichter 111, der im Falle der parallelen Anordnung der beiden Atemgasverdichter 111, 300 gemäß Figur 5c erzeugt wird, entfällt, weil der gepulste Atemgasverdichter 111 geplant oder ungeplant außer Betrieb ist.

Grundsätzlich können die beiden Atemgasverdichter 111, 300 können unabhängig voneinander den Patienten selbstständig mit einem Atemgas-Druckstrom 112 versorgen, sodass durch die redundante Anordnung der Atemgasverdichter 111, 300 die Zuverlässigkeit der Beatmungsmaschine 100 gesteigert ist.

### Bezugszeichenliste

- 100: Beatmungsmaschine
- 101: Überwachungseinheit
- 102: Inspirationsschenkel
- 103: Expirationsschenkel
- 104: gemeinsamer Inspirations- und Expirationsschenkel
- 105: Patientenventil
- 106: Lunge oder Patient
- 107: Atemgasstrom
- 108: Ansaugstutzen für Umgebungsluft
- 109: Ansaugstutzen für Sauerstoff
- 110: Mischeinheit
- 111: (gepulste) Überdruckeinheit, insbesondere Atemgasverdichter
- 112: Atemgas-Druckstrom
- 113: elektronische Einheit
- 114: Messsignaleingang
- 115: Steuersignalausgang
- 116: Mittel zur Signalübertragung
- 117: Befeuchtungseinheiten
- 118: Filter
- 119: Absperr- oder Magnetventile
- 120: Durchflusssensor
- 121: Sensoreinheit des Inspirationsschenkels
- 121f: Durchflusssensor
- 121o: Sauerstoffsensor
- 121p: Drucksensor
- 122: Sensoreinheit des Expirationsschenkels
- 122f: Durchflusssensor
- 122p: Drucksensor
- 123: Sensoreinheit des gemeinsamen Inspirations- und Expirationsschenkels
- 123f: Durchflusssensor
- 124: Einwegeventile
- 200: Tidalvolumen
- 201: Dauer des Einatmens
- 202: Anlaufzeit
- 203: Atemgasvolumenstrom
- 204: voreingestellter Inspirationsflow
- 205: applizierter Inspirationflow
- 300: (konstante) Überdruckeinheit, insbesondere Atemgasverdichter
- 301: Abzweig
- 302: Bypassleitung
- 303: Dreiwegeventil
- 304: Vierwegeventil
- 305: Ausblasstutzen
- 306: Durchflusssensor
- 307: erster Mischeinheitausgang
- 308: zweiter Mischeinheitausgang
- 309: der seriellen Schaltung zugeordnete Einmündung
- 310: der parallelen Schaltung zugeordnete Einmündung

## Patentansprüche

1. Beatmungsmaschine (100) zur Beatmung eines Patienten, wobei die Beatmungsmaschine (100) mindestens eine Überdruckeinheit (300), insbesondere einen Atemgasverdichter, zum Bereitstellen eines Atemgas-Druckstroms (112) in einem oder für einen Inspirationskanal (102) und ein oder mehrere, die Überdruckeinheit (300) kontrollierende, elektronische Steuerungsmittel (MCU) aufweist,
**wobei**
das oder die Steuerungsmittel (MCU) programm- und/oder schaltungstechnisch dazu eingerichtet ist, die Überdruckeinheit (300) mit ihrem Atemgas-Druckstrom (112) strömungstechnisch dem Inspirationskanal (102) selektiv zu wenigstens einer vorab festgelegten Zeit- und/oder Triggerbedingung zuzuschalten und/oder von dem Inspirationskanal (102) zu trennen,
**dadurch gekennzeichnet, dass**
zusätzlich zu der konstanten Überdruckeinheit (300) eine weitere Überdruckeinheit (111) als pulsierende Überdruckeinheit angeordnet ist, die vorzugsweise von dem oder den Steuermitteln (MCU) zum Bereitstellen eines Atemgas-Druckstroms (112) angesteuert oder ansteuerbar ist, der zwischen einem höheren, eine Überdruck- Beatmung oder Hyperventilation ermöglichenden Druckbereich zu einem niedrigeren, eine Exspiration bzw. Ausatmung ermöglichenden Druckbereich alterniert.

2. Beatmungsmaschine nach Anspruch 1,
**dadurch gekennzeichnet, dass**
das oder die Steuerungsmittel (MCU) programm- und/oder schaltungstechnisch dazu eingerichtet sind, die Überdruckeinheit (300) als konstante Überdruckeinheit zum Bereitstellen eines Atemgas-Druckstroms (112), der konstant ist oder ständig in einem höheren Druckbereich liegt, anzusteuern oder zu betreiben, und der höhere Druckbereich einer Überdruck-Beatmung, Inspiration und/oder Hyper-Ventilation beim Patienten entspricht.

3. Beatmungsmaschine (100) nach Anspruch 1 oder 2,
**dadurch gekennzeichnet, dass**
das oder die Steuerungsmittel (MCU) dazu eingerichtet sind, die Triggerbedingung über ein oder mehrere Messignale einer oder mehrerer Sensoreinheiten (121, 122, 123) abzuleiten und davon abhängig die Überdruckeinheit (300) anzusteuern.

4. Beatmungsmaschine (100) nach Anspruch 3,
**dadurch gekennzeichnet, dass**
mindestens eine Sensoreinheit (123) zum Feststellen der Triggerbedingung in einem gemeinsamen oder gleichzeitigen Inspirations- und Exspirationsschenkel (104) der Beatmungsmaschine (100) angeordnet ist.

5. Beatmungsmaschine (100) nach Anspruch 3 oder 4,
**dadurch gekennzeichnet, dass**
die einen oder mehreren Sensoreinheiten (121, 122, 123) zum Feststellen der Triggerbedingung einen Drucksensor (121p,122p) und/oder einen Durchflusssensor (121f,122f,123f) und/oder einen Sauerstoffsensor (121o) aufweisen.

6. Beatmungsmaschine (100) nach einem der vorangehenden Ansprüche, **dadurch gekennzeichnet, dass**
die konstante Überdruckeinheit (300) als Atemgasverdichter, bspw. Rotationsgebläse oder Hubkolbenverdichter, ausgebildet und von dem oder den Steuerungsmittel ansteuerbar ist, um druckseitig oder ausgangsseitig einen vorab festlegbaren Überdruck bereitzustellen, der konstant oder ständig in dem höheren Druckbereich liegt.

7. Beatmungsmaschine (100) nach Anspruch 6, wobei der Atemgasverdichter (300) per Ansteuerung durch das oder die Steuerungsmittel (MCU) zum Ansaugen von Atemgas (107) eingangsseitig oder saugseitig mit einer Mischeinheit (110) verbunden oder verbindbar ist,
**dadurch gekennzeichnet, dass**
der Atemgasverdichter (300) zum Ansaugen von Umgebungsluft eingangsseitig oder saugseitig mit der Umgebung und/oder über eine der Umgehung der Mischeinheit (110) dienende Bypassleitung (301) mit einem Ansaugstutzen (108) der Mischeinheit und/oder der Beatmungsmaschine (100) verbunden oder verbindbar ist.

8. Beatmungsmaschine (100) nach einem der vorangehenden Ansprüche,
**dadurch gekennzeichnet, dass**
die konstante Überdruckeinheit (300) zum Bereitstellen eines pulsierenden Atemgas-Druckstroms (112) und die pulsierende Überdruckeinheit (111) zum Bereitstellen eines konstanten Atemgas-Druckstroms (112) ausgebildet oder angesteuert oder ausbildbar oder ansteuerbar ist.

9. Beatmungsmaschine (100) nach einem der vorangehenden Ansprüche,
**dadurch gekennzeichnet, dass**
die pulsierende Überdruckeinheit (111) und die konstante Überdruckeinheit (300) strömungstechnisch in Reihe angeordnet oder anordenbar sind, wobei vorzugsweise die konstante Überdruckeinheit (300) eingangsseitig oder saugseitig der pulsierenden Überdruckeinheit (111) angeordnet oder anordenbar ist.

10. Beatmungsmaschine (100) nach einem der vorangehenden Ansprüche,
**dadurch gekennzeichnet, dass**
die pulsierende Überdruckeinheit (111) und die konstante Überdruckeinheit (300) strömungstechnisch parallel angeordnet oder anordenbar sind.

11. Beatmungsmaschine (100) nach einem der Ansprüche 2-10,
**dadurch gekennzeichnet, dass**
sie in einem ersten Betriebsmodus zum Bereitstellen eines pulsierenden Atemgas-Druckstroms (112) durch die pulsierenden Überdruckeinheit (111) ausgebildet ist, und
in einem zweiten Betriebsmodus zum Bereitstellen eines konstanten Atemgas-Druckstroms (112) oder eines Atemgas-Druckstroms (112) konstant in dem höheren Druckbereich jeweils durch die konstante Überdruckeinheit (300) ausgebildet ist.

12. Beatmungsmaschine (100) nach Anspruch 11,
**dadurch gekennzeichnet, dass**
in dem zweiten Betriebsmodus die Beatmungsmaschine (100) zum Bereitstellen eines pulsierenden Atemgas-Druckstroms (112) durch die pulsierende Überdruckeinheit (111) ausgebildet ist.

13. Beatmungsmaschine (100) nach Anspruch 11,
**dadurch gekennzeichnet, dass**
der erste Betriebsmodus und der zweite Betriebsmodus während eines Atemzugs des Patienten aktivierbar sind, vorzugsweise wenn das Steuerungsmittel (MCU) die Triggerbedingung erkennt.

14. Beatmungsmaschine (100) nach einem der Ansprüche 11 oder 13,
**dadurch gekennzeichnet, dass**
das oder die Steuermittel dazu eingerichtet sind, in dem zweiten Betriebsmodus die konstante Überdruckeinheit (300) zur Bereitstellung eines konstanten Atemgas-Druckstroms (112) oder eines Atemgas-Druckstroms (112) konstant oder ständig in dem höheren Druckbereich anzusteuern, bis der durch die pulsierende Überdruckeinheit (111) bereitgestellte, pulsierende Atemgas-Druckstrom (112) einen vorab festgelegten Atemgasvolumenstrom (203,204) und/oder einen vorab festgelegten Gasdruck erreicht.

## Claims

1. A breathing apparatus (100) for artificially ventilating a patient, wherein the breathing apparatus (100) has at least one overpressure unit (300), in particular a compressor for respiratory gas, for providing a respiratory gas pressure flow (112) in or for an inhalation channel (102), and one or more electronic control means (MCU) controlling the overpressure unit (300),
**wherein**
the control means (MCU) is set up, in terms of programming and/or circuitry, to connect the overpressure unit (300) with its respiratory gas pressure flow (112) selectively, fluidically, to the inhalation channel (102) at at least one previously determined time and/or trigger condition and/or separate it from the inhalation channel (102),
**characterised in that**
in addition to the continuous overpressure unit (300), a further overpressure unit (111) is arranged as pulsating overpressure unit which preferably is or can be controlled by the control means (MCU) for providing a respiratory gas pressure flow (112) which alternates between a higher pressure range making possible overpressure inhalation or hyperventilation and a lower pressure range making possible expiration or exhalation.

2. The breathing apparatus according to claim 1,
**characterised in that**
the control means (MCU) is set up, in terms of programming and/or circuitry, to control or operate the overpressure unit (300) as continuous overpressure unit for providing a respiratory gas pressure flow (112) which is continuous or is permanently in a higher pressure range, and the higher pressure range corresponds to an overpressure ventilation, inhalation and/or hyperventilation in the patient.

3. The breathing apparatus (100) according to claim 1 or 2,
**characterised in that**
the control means (MCU) is set up to derive the trigger condition via one or more measurement signals of one or more sensor units (121, 122, 123), and to control the overpressure unit (300) dependent thereon.

4. The breathing apparatus (100) according to claim 3,
**characterised in that**
at least one sensor unit (123) for establishing the trigger condition is arranged in a common or simultaneous inhalation and expiration tube (104) of the breathing apparatus (100).

5. The breathing apparatus (100) according to claim 3 or 4,
**characterised in that**
the one or more sensor units (121, 122, 123) for establishing the trigger condition have a pressure sensor (121p, 122p) and/or a flow sensor (121f, 122f, 123f) and/or an oxygen sensor (121o).

6. The breathing apparatus (100) according to one of the preceding claims,
**characterised in that**
the continuous overpressure unit (300) is designed as a compressor for respiratory gas, e.g. as a rotary fan or reciprocating compressor, and can be controlled by the control means, in order to provide an overpressure which can be established in advance, pressure side or output side, which overpressure lies continuously or permanently in the higher pressure range.

7. The breathing apparatus (100) according to claim 6, wherein the compressor for respiratory gas (300) is or can be connected to a mixing unit (110) for drawing in respiratory gas (107) at the input side or suction side, by being controlled by the control means (MCU),
**characterised in that**
to draw in ambient air, the compressor for respiratory gas (300) is or can be connected, input side or suction side, to the environment and/or to an intake socket (108) of the mixing unit and/or of the breathing apparatus (100) via a bypass line (301) serving to circumvent the mixing unit (110).

8. The breathing apparatus (100) according to one of the preceding claims,
**characterised in that**
the continuous overpressure unit (300) is or can be designed, or is or can be controlled, for providing a pulsating respiratory gas pressure flow (112) and the pulsating overpressure unit (111) is or can be designed, or is or can be controlled, for providing a continuous respiratory gas pressure flow (112).

9. The breathing apparatus (100) according to one of the preceding claims,
**characterised in that**
the pulsating overpressure unit (111) and the continuous overpressure unit (300) are or can be arranged, fluidically, in series, wherein preferably the continuous overpressure unit (300) is or can be arranged input side or suction side of the pulsating overpressure unit (111).

10. The breathing apparatus (100) according to one of the preceding claims,
**characterised in that**
the pulsating overpressure unit (111) and the continuous overpressure unit (300) are or can be arranged, fluidically, parallel.

11. The breathing apparatus (100) according to one of claims 2-10,
**characterised in that**
it is designed, in a first mode of operation, for providing a pulsating respiratory gas pressure flow (112) through the pulsating overpressure unit (111), and
is designed, in a second mode of operation, for providing a continuous respiratory gas pressure flow (112) or a respiratory gas pressure flow (112) continuously in the higher pressure range, each through the continuous overpressure unit (300).

12. The breathing apparatus (100) according to claim 11,
**characterised in that**
in the second mode of operation, the breathing apparatus (100) is designed for providing a pulsating respiratory gas pressure flow (112) through the pulsating overpressure unit (111).

13. The breathing apparatus (100) according to claim 11,
**characterised in that**
the first mode of operation and the second mode of operation can be activated while a patient is drawing breath, preferably when the control means (MCU) recognises the trigger condition.

14. The breathing apparatus (100) according to one of claims 11 or 13,
**characterised in that**
in the second mode of operation, the control means are designed to control the continuous overpressure unit (300) for providing a continuous respiratory gas pressure flow (112) or to actuate a respiratory gas pressure flow (112) continuously or permanently in the higher pressure range until the pulsating respiratory gas pressure flow (112) provided by the pulsating overpressure unit (111) reaches a gas flow (203, 204) established in advance and/or a gas pressure established in advance.

## Revendications

1. Respirateur (100) pour la ventilation d'un patient,
dans lequel le respirateur (100) comporte au moins une unité de surpression (300), en particulier un compresseur de gaz respiratoire, pour fournir un courant sous pression de gaz respiratoire (112) dans ou pour un canal d'inhalation (102) et un ou plusieurs moyens de commande électroniques (MCU) commandant l'unité de surpression (300),
dans lequel
le ou les moyens de commande (MCU) sont conçus, par programme et/ou par circuit, pour relier l'unité de surpression (300) avec son courant sous pression de gaz respiratoire (112) au canal d'inhalation (102) et/ou pour la séparer du canal d'inhalation (102) de manière sélective conformément à au moins une condition de temps et/ou de déclenchement établie à l'avance, **caractérisé en ce que**,
outre l'unité de surpression constante (300), une autre unité de surpression (111) est disposée en tant qu'unité de surpression pulsée, qui est de préférence commandée ou peut être commandée par le ou les moyens de commande (MCU) pour fournir un courant sous pression de gaz respiratoire (112) qui alterne entre une plage de pression plus élevée permettant une respiration en surpression ou une hyperventilation et une plage de pression plus basse permettant une expiration.

2. Respirateur selon la revendication 1, **caractérisé en ce que**
le ou les moyens de commande (MCU) sont conçus par programme et/ou par circuit pour commander ou faire fonctionner l'unité de surpression (300) en tant qu'unité de surpression constante pour fournir un courant sous pression de gaz respiratoire (112) qui est constant ou se situe en permanence dans une plage de pression plus élevée, et la plage de pression plus élevée correspond à une respiration en surpression, une inhalation et/ou une hyperventilation chez le patient.

3. Respirateur (100) selon la revendication 1 ou 2, **caractérisé en ce que**
le ou les moyens de commande (MCU) sont conçus pour déduire la condition de déclenchement par le biais d'un ou de plusieurs signaux de mesure provenant d'une ou de plusieurs unités de détection (121, 122, 123) et pour commander l'unité de surpression (300) en fonction de ceux-ci.

4. Respirateur (100) selon la revendication 3, **caractérisé en ce qu'**au moins une unité de détection (123) destinée à détecter la condition de déclenchement est disposée dans une branche d'inhalation et d'expiration (104) commune ou simultanée du respirateur (100) .

5. Respirateur (100) selon la revendication 3 ou 4, **caractérisé en ce que**
lesdites une ou plusieurs unités de détection (121, 122, 123) destinées à détecter la condition de déclenchement comprennent un capteur de pression (121p, 122p) et/ou un capteur de débit (121f, 122f, 123f) et/ou un capteur d'oxygène (121o).

6. Respirateur (100) selon l'une des revendications précédentes, **caractérisé en ce que**
l'unité de surpression constante (300) est réalisée sous la forme d'un compresseur de gaz respiratoire, par exemple un ventilateur rotatif ou un compresseur à piston alternatif, et peut être commandée par le ou les moyens de commande pour fournir, côté pression ou côté sortie, une surpression pouvant être établie à l'avance, qui se situe de manière constante ou permanente dans la plage de pression plus élevée.

7. Respirateur (100) selon la revendication 6, dans lequel le compresseur de gaz respiratoire (300) est relié ou peut être relié à une unité de mélange (110) par commande par le ou les moyens de commande (MCU) pour aspirer du gaz respiratoire (107) côté entrée ou côté aspiration,
**caractérisé en ce que**
le compresseur de gaz respiratoire (300) est relié ou peut être relié à une tubulure d'aspiration (108) de l'unité de mélange et/ou du respirateur (100) pour aspirer de l'air ambiant côté entrée ou côté aspiration avec l'environnement et/ou par une conduite de dérivation (301) servant à contourner l'unité de mélange (110).

8. Respirateur (100) selon l'une des revendications précédentes, **caractérisé en ce que**
l'unité de surpression constante (300) est conçue ou commandée ou peut être conçue ou commandée pour fournir un courant sous pression de gaz respiratoire (112) pulsé et l'unité de surpression pulsée (111) est conçue ou peut être conçue pour fournir un courant sous pression de gaz respiratoire (112) constant.

9. Respirateur (100) selon l'une des revendications précédentes, **caractérisé en ce que**
l'unité de surpression pulsée (111) et l'unité de surpression constante (300) sont disposées ou peuvent être disposées en série du point de vue de la technique d'écoulement, l'unité de surpression constante (300) étant de préférence disposée ou pouvant être disposée du côté entrée ou du côté aspiration de l'unité de surpression pulsée (111).

10. Respirateur (100) selon l'une des revendications précédentes, **caractérisé en ce que**
l'unité de surpression pulsée (111) et l'unité de surpression constante (300) sont disposées ou peuvent être disposées en parallèle du point de vue de la technique d'écoulement.

11. Respirateur (100) selon l'une des revendications 2-10, **caractérisé en ce que**,
dans un premier mode de fonctionnement, celui-ci est conçu pour fournir un courant sous pression de gaz respiratoire (112) pulsé par le biais de l'unité de surpression pulsée (111), et
dans un deuxième mode de fonctionnement, pour fournir respectivement un courant sous pression de gaz respiratoire (112) constant ou un courant sous pression de gaz respiratoire (112) qui est constant dans la plage de pression plus élevée, au moyen de l'unité de surpression constante (300).

12. Respirateur (100) selon la revendication 11, **caractérisé en ce que**,
dans le second mode de fonctionnement, le respirateur (100) est conçu pour fournir un courant sous pression de gaz respiratoire (112) pulsé au moyen de l'unité de surpression pulsée (111).

13. Respirateur (100) selon la revendication 11, **caractérisé en ce que**
le premier mode de fonctionnement et le second mode de fonctionnement peuvent être activés pendant une respiration du patient, de préférence lorsque le moyen de commande (MCU) détecte la condition de déclenchement.

14. Respirateur (100) selon l'une des revendications 11 ou 13, **caractérisé en ce que**
le ou les moyens de commande sont conçus pour commander, dans le second mode de fonctionnement, l'unité de surpression constante (300) afin de fournir un courant sous pression de gaz respiratoire (112) constant ou un courant sous pression de gaz respiratoire (112) qui est constant ou permanent dans la plage de pression plus élevée, jusqu'à ce que le courant sous pression de gaz respiratoire (112) pulsé fourni par l'unité de surpression pulsée (111) atteigne un débit volumique de gaz respiratoire (203, 204) établi à l'avance et/ou une pression de gaz établie à l'avance.
